# EUROPÄISCHE PATENTANMELDUNG

(11) **EP 1 378 742 A2**
(43) Veröffentlichungstag der Anmeldung: **07.01.2004**
(21) Anmeldenummer: 03013901.8
(22) Anmeldetag: 20.06.2003
(51) Int. Cl.: G01N 1/20, A01D 41/127

(54) **Vorrichtung zur Entnahme von Proben**

(30) Priorität: 06.07.2002 DE 10230475
(71) Anmelder: DEERE & COMPANY, Moline, Illinois 61265-8098 (US)
(72) Erfinder: Kormann, Georg, Dr., 66424 Homburg (DE); Flohr, Werner, 67633 Kaiserslautern (DE)
(74) Vertreter: Holst, Sönke, Dr.

(57) **Zusammenfassung**

Die Erfindung bezieht sich auf eine Vorrichtung zur Entnahme von Proben aus einem in einem Förderkanal strömenden Erntegutstrom in einer Erntemaschine (10).

Es wird ein Leitelement (32) vorgeschlagen, das einen in den Förderkanal einführbaren Bereich umfasst. Dadurch wird das Erntegut zur Probengewinnung zwangsumgeleitet.

## Beschreibung

Die Erfindung betrifft eine Vorrichtung zur Entnahme von Proben aus einem in einem Förderkanal strömenden Erntegutstrom in einer Erntemaschine.

In der Landwirtschaft besteht ein Interesse an Informationen über Qualitätsparameter von Erntegut. Einige Parameter des Ernteguts, wie die Feuchtigkeit, können bereits während des Erntevorgangs erfasst werden, wie z. B. in der EP 0 908 086 A anhand eines Mähdreschers beschrieben wird. Zur Ermittlung mancher anderer Parameter des Ernteguts, wie dem Stärkegehalt, ist eine Entnahme von Proben zur späteren Analyse in einem Labor sinnvoll. Dabei ist eine Automatisierung wünschenswert.

In der FR 2 801 380 A wird eine automatische Probenentnahmeeinrichtung für einen Mähdrescher beschrieben. Im Körnerelevator ist eine verstell- und schließbare Öffnung vorgesehen, durch die Körner zu einem Förderer rieseln, der sie in einen Schlauch füllt. Durch Abklemmen von Schlauchabschnitten werden einzelne Proben erzeugt, deren Entstehungsort durch ein satellitengestütztes Positionserfassungssystem erfasst wird. In einer Datei wird zur späteren Identifikation eine Information über die Position und die Probennummer abgelegt.

Gemäß der Offenbarungen der EP 0 908 086 A und der FR 2 801 380 A rieselt ein Teil des Erntegutstroms durch eine Öffnung hindurch und wird dann gesammelt, bis eine hinreichende Menge als Probe zur Verfügung steht. Da das Material aufgrund seiner Schwerkraft aus dem Gutstrom entnommen wird, können Probleme bei hohen Gutfeuchten auftreten. Für die Entnahme von Silage aus dem Materialstrom eines Feldhäckslers sind diese Lösungen ungeeignet.

Das der Erfindung zu Grunde liegende Problem wird darin gesehen, eine verbesserte Probenentnahmevorrichtung zur Verfügung zu stellen.

Dieses Problem wird erfindungsgemäß durch die Lehre des Patentanspruchs 1 gelöst, wobei in den weiteren Patentansprüchen Merkmale aufgeführt sind, die die Lösung in vorteilhafter Weise weiterentwickeln.

Auf diese Weise wird ein Bereich des Leitelements in den Erntegutstrom eingebracht, was dazu führt, dass das Erntegut zwangsumgeleitet wird. Der dadurch entstehende Aufstau stellt sicher, dass tatsächlich Erntegut aus dem Förderkanal entnommen wird. Durch eine geeignete Wahl der Größe des Leitelements kann sichergestellt werden, dass eine repräsentative Probe über den gesamten Querschnitt des Gutflusses entnommen werden kann.

Es bietet sich an, das Leitelement schwenkbar anzulenken. Dadurch kann es zwischen einer Probenentnahmeposition und einer Außerbetriebsposition bewegt werden. In der Außerbetriebsposition verschließt es vorzugsweise eine Öffnung in einer Wand des Förderkanals, so dass ein ungestörter Erntegutstrom möglich ist. In der Probenentnahmeposition gibt es die Öffnung frei und ein Bereich des Leitelements erstreckt sich in den Erntegutstrom hinein. Das umgeleitete Erntegut strömt dann in der Regel durch die Öffnung hindurch. Die Schwenkachse des Leitelements erstreckt sich zweckmäßigerweise zumindest etwa quer zur Gutflussrichtung. Es sind auch Ausführungsformen mit einem (ausschließlich oder zusätzlich) verschiebbaren Leitelement denkbar, das in den Förderkanal hineingeschoben und daraus herausgezogen werden kann.

Grundsätzlich wäre denkbar, das Leitelement an einem Ende schwenkbar an der Wand des Förderkanals anzulenken. Besonders zweckmäßig ist aber die Anordnung der Schwenkachse (bezüglich der Flussrichtung des Ernteguts im Förderkanal) etwa in der Mitte des Leitelements. Dadurch kann das Leitelement von zwei Seiten benutzt werden. Ist die eine Seite nach längerem Gebrauch abgeschliffen, wird das Leitelement umgedreht, bzw. beide Seiten werden abwechselnd und somit langsamer abgenutzt. Außerdem besteht die Möglichkeit, einen rotativen Antrieb mit nur einer Drehrichtung zu verwenden.

Bezüglich der Positionierung des in den Förderkanal einbringbaren Bereichs des Leitelements bestehen verschiedene Möglichkeiten. In einer ersten Ausführungsform ist dieser Bereich gegenüber der Flussrichtung des Ernteguts in einem Winkel von weniger als 90° angeordnet. Bei einem schwenkbaren Leitelement ist er dann stromauf der Schwenkachse des Leitelements angeordnet. Das Gut behält somit auch dann, wenn sich das Leitelement in der Probenentnahmeposition befindet, seine grundsätzliche Flussrichtung wenigstens näherungsweise bei, es wird jedoch um einen bestimmten Winkel umgelenkt. In einer anderen Ausführungsform ist der sich in den Förderkanal erstreckende Bereich des Leitelements in der Probenentnahmeposition quer zur Flussrichtung des Ernteguts orientiert oder der Winkel zwischen dem Leitelement und der Flussrichtung ist sogar größer als 90°. Bei einem schwenkbaren Leitelement befindet sich der genannte Bereich dann stromab der Schwenkachse des Leitelements. Es bildet sich ein Stau des Ernteguts, der dazu führt, dass Erntegut zur Gewinnung der Probe aus dem Förderkanal heraus gelangt.

Um auf größeren Feldern eine höhere Anzahl an Proben zu entnehmen, bietet sich eine Automatisierung der Probenentnahme an. Dazu sind ein fremdkraftbetätigter Antrieb zum Schwenken des Leitelements um dessen Schwenkachse und geeignete Mittel zum Einfügen des aus dem Erntegutstrom entnommenen Ernteguts in einen Probenbehälter zweckmäßig. Eine geeignete Steuerung kontrolliert den Antrieb, der auf eine manuelle Eingabe hin oder beim Erreichen vorbestimmter zeitlich und/oder örtlich definierter Punkte veranlasst, dass das Leitelement in den Förderkanal verschwenkt wird. Das abgezweigte Erntegut gelangt durch einen Förderer bzw. seine eigene kinetische Energie in einen Probenbehälter. Die aktuelle Position des Leitelements kann durch geeignete Sensoren überwacht werden. Sie stellen sicher, dass die Probe tatsächlich gezogen wurde und dass das Leitelement anschließend wieder in seine Außerbetriebsposition zurückgefahren ist.

Vorzugsweise wird z. B. in einer Datenbank eine Information abgelegt, anhand der der jeweilige Probenbehälter später identifiziert werden kann. Es ist beispielsweise ein Bedrucken des Probenbehälters mit einer entsprechenden Information denkbar, z. B. einem Barcode, oder das Einspeichern der Information in einen mit dem Behälter verbundenen Transponder. Denkbar wäre auch, in einer Datenbank eine Probenbehälternummer bzw. Information über die Position abzulegen, an der sich der Probenbehälter befindet. Auch kann der mittels eines - vorzugsweise satellitengestützt arbeitenden - Positionserfassungssystems ermittelte Entstehungsort der Probe (oder eine andere Größe, anhand der die Probe später identifizierbar ist, wie die Erzeugungszeit) abgespeichert bzw. auf den Probenbehälter gedruckt werden. Anstelle die Probe in einem Probenbehälter zu lagern und später in einem Labor zu analysieren, wäre aber auch mittels geeigneter Geräte eine Analyse vor Ort möglich.

Die Erfindung eignet sich für jegliche Erntemaschinen, bei denen das Erntegut einen Förderkanal durchströmt, z. B. Ballenpressen, Ladewagen, Mähdrescher oder Feldhäcksler. Bei letzteren wird die Leiteinrichtung vorzugsweise im Austragschacht angebracht.

In der Zeichnung sind zwei nachfolgend näher beschriebene Ausführungsbeispiele der Erfindung dargestellt. Es zeigt:
- Fig. 1: eine Erntemaschine in Seitenansicht und in schematischer Darstellung,
- Fig. 2: den Austragschacht der Erntemaschine mit einer ersten Ausführungsform eines daran angebrachten Leitelements zur Probenentnahme, und
- Fig. 3: den Austragschacht der Erntemaschine mit einer zweiten Ausführungsform eines daran angebrachten Leitelements zur Probenentnahme.

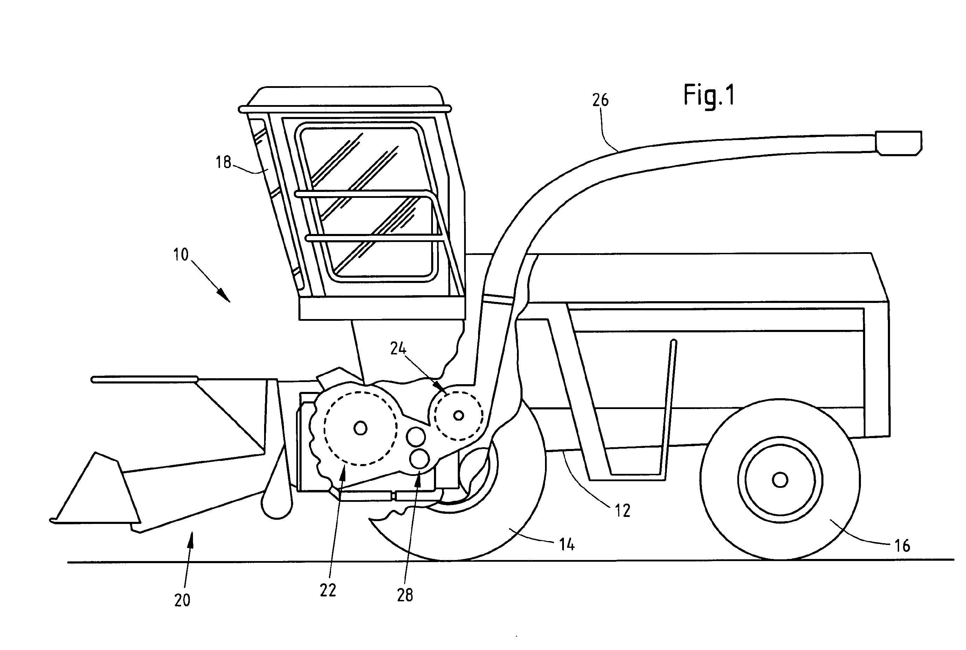

Eine in Figur 1 gezeigte Erntemaschine 10 in der Art eines selbstfahrenden Feldhäckslers baut sich auf einem Rahmen 12 auf, der von vorderen und rückwärtigen Rädern 14 und 16 getragen wird. Die Bedienung der Erntemaschine 10 erfolgt von einer Fahrerkabine 18 aus, von der aus eine Erntegutaufnahmevorrichtung 20 einsehbar ist. Mittels der Erntegutaufnahmevorrichtung 20 vom Boden aufgenommenes Gut, z. B. Mais, Gras oder dergleichen wird einer Häckseltrommel 22 zugeführt, die es in kleine Stücke häckselt und es einer Fördervorrichtung 24 aufgibt. Das Gut verlässt die Erntemaschine 10 zu einem nebenher fahrenden Anhänger über einen drehbaren Austragsschacht 26. Zwischen der Häckseltrommel 22 und der Fördervorrichtung 24 erstreckt sich eine Nachzerkleinerungsvorrichtung 28, durch die das zu fördernde Gut der Fördervorrichtung 24 tangential zugeführt wird.

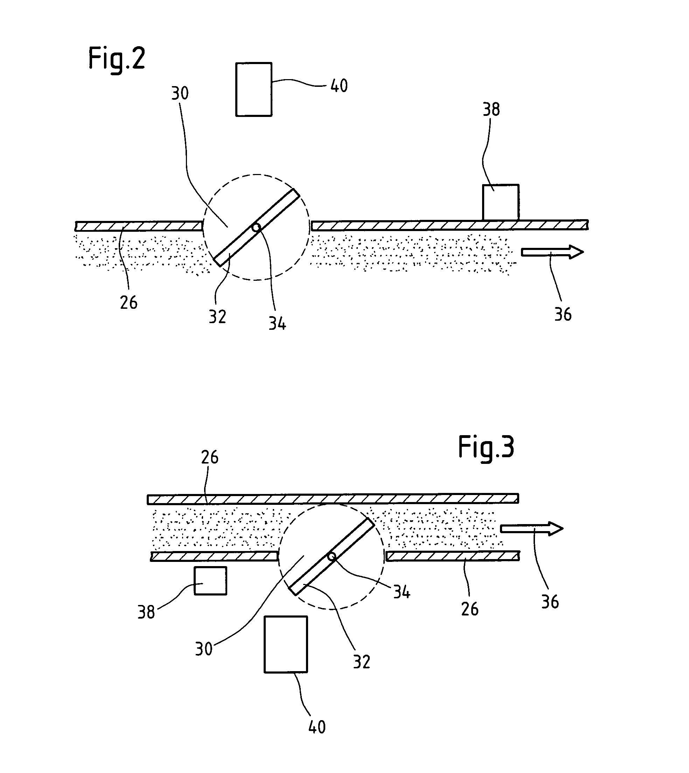

In der Figur 2 ist ein vertikaler Querschnitt entlang des Austragschachts 26 dargestellt. In der oberen Wand des Austragschachts 26 ist eine Öffnung 30 vorgesehen. Innerhalb der Öffnung 30 befindet sich ein Leitelement 32 in Form eines flachen Blechs, das um eine sich horizontal und quer zur Zeichenebene erstreckende Schwenkachse 34 schwenkbar gelagert ist. Das Leitelement 32 kann in Draufsicht kreisförmig oder rechteckig sein. Die Schwenkachse erstreckt sich bezüglich der Flussrichtung des Erntegut, die durch den Pfeil 36 gekennzeichnet wird, durch die Mitte des Leitelements 32. Ein fremdkraftbetätigter Antrieb 38 in Form eines Elektro- bzw. Hydraulikmotors ermöglicht über nicht dargestellte Übertragungselemente eine Drehung des Leitelements 32 um die Schwenkachse 34. In der Figur 2 ist das Leitelement 32 in seiner Probenentnahmeposition dargestellt, in der das den Austragschacht 26 durchströmende Erntegut unter einem Winkel von etwa 45° auf den Bereich des Leitelements 32 prallt, der sich (bezüglich des Gutflusses) stromauf der Schwenkachse 34 befindet, und durch das Leitelement 32 nach oben abgelenkt wird, sodass es in einen Probenbehälter 40 gelangt. Mittels des Antriebs 38 kann das Leitelement 32 in eine Außerbetriebsposition verbracht werden, in der es sich parallel zur benachbarten Wand des Austragschachts 26 erstreckt und die Öffnung 30 verschließt. Der Probenbehälter 40, bei dem es sich z. B. um eine Flasche, Tüte oder Dose handeln kann, wird durch eine nicht eingezeichnete Einrichtung, z. B. einen Greifarm, aus einem Magazin entnommen und nachdem er mit der Erntegutprobe gefüllt ist, wieder dort abgelegt. Eine elektronische Steuerung ordnet für spätere Auswertungszwecke seinen Platz im Magazin dem Ort zu, an dem die Probe entnommen wurde. Auch die Verwendung eines Schlauchs, wie in der FR 2 801 380 A vorgeschlagen, wäre zur Aufnahme der Proben denkbar.

In der Figur 3 ist eine zweite Ausführungsform eines Leitelements 32 dargestellt. Der ersten Ausführungsform entsprechende Elemente sind mit übereinstimmenden Bezugszeichen versehen. In der Figur 3 ist jedoch ein horizontaler Querschnitt entlang des Austragschachts 26 wiedergegeben. Der Austragschacht umfasst die beiden in Figur 2 dargestellten seitlichen Wände. In einer von ihnen ist die Öffnung 30 für das Leitelement 32 vorgesehen, das um die sich vertikal erstreckende Schwenkachse 34 schwenkbar ist. In der dargestellten Probenentnahmeposition ist der sich in das Innere des Austragschachts 26 erstreckende Bereich des Leitelements 32 bezüglich des Erntegutflusses stromab der Schwenkachse 34 angeordnet. Das Erntegut trifft unter einem Winkel von etwa 135° auf das Leitelement 32. Es bildet sich ein Rückstau, der dazu führt, dass das Erntegut in den Probenbehälter 40 gelangt. Auch bei dieser Ausführungsform ist der Antrieb 38 eingerichtet, das Leitelement 32 in eine Außerbetriebsposition zu verbringen, in der es sich parallel zur benachbarten Wand des Austragschachts 26 erstreckt und die Öffnung 30 verschließt.

Die dargestellte erfindungsgemäße Vorrichtung ermöglicht eine automatisierte Entnahme von Proben aus dem Austragschacht 26 der Erntemaschine 10 in Form eines Feldhäckslers. Diese Proben sind von grundlegender Bedeutung für die Entwicklung von Kalibrierungen von NIR-Messsystemen. Zudem wird eine georeferenzierte Probennahme bei der Ernte von Streifentests möglich. Weiterhin wird dem Besitzer die Möglichkeit gegeben, ein Feuchtigkeitsmesssystem oder ein Kontrollsystem für Qualitätsparameter des Ernteguts auf seine Genauigkeit zu überprüfen und gegebenenfalls neu zu kalibrieren.

## Patentansprüche

1. Vorrichtung zur Entnahme von Proben aus einem in einem Förderkanal strömenden Erntegutstrom in einer Erntemaschine (10), **gekennzeichnet durch** ein Leitelement (32), das einen in den Förderkanal einführbaren Bereich umfasst.

2. Vorrichtung nach Anspruch 1, **dadurch gekennzeichnet, dass** das Leitelement (32) verschwenkbar ist.

3. Vorrichtung nach Anspruch 1 oder 2, **dadurch gekennzeichnet, dass** das Leitelement (32) zwischen einer Probenentnahmeposition, in der es eine Öffnung (30) in einer Wand des Förderkanals freigibt und in der sich ein Bereich des Leitelements (32) im Förderkanal befindet, und einer Ausserbetriebsposition beweglich ist, in der es die Öffnung (30) in der Wand verschließt.

4. Vorrichtung nach Anspruch 3, **dadurch gekennzeichnet, dass** das Erntegut bei in die Probenentnahmeposition verbrachtem Leitelement (32) die Öffnung (30) in der Wand durchläuft.

5. Vorrichtung nach einem der Ansprüche 2 bis 4, **dadurch gekennzeichnet, dass** sich die Schwenkachse (34) des Leitelements (32) zumindest näherungsweise quer zur Gutflussrichtung erstreckt.

6. Vorrichtung nach einem der Ansprüche 2 bis 5, **dadurch gekennzeichnet, dass** sich die Schwenkachse (34) des Leitelements (32) zumindest näherungsweise in der Mitte von dessen in Gutflussrichtung gemessener Längserstreckung befindet.

7. Vorrichtung nach einem der Ansprüche 1 bis 6, **dadurch gekennzeichnet, dass** der sich in den Förderkanal erstreckende Bereich des Leitelements (32) in der Probenentnahmeposition gegenüber der Flussrichtung des Ernteguts um weniger oder mehr als 90° geneigt ist.

8. Vorrichtung nach einem der vorhergehenden Ansprüche, **gekennzeichnet durch** einen fremdkraftbetätigten Antrieb (38), der zum Schwenken des Leitelements (32) um dessen Schwenkachse eingerichtet ist, **durch** Mittel zum Einfügen von Erntegut, das mittels des Leitelements aus dem Erntegutstrom entnommen wurde, in einen Probenbehälter (40) und **durch** Mittel zum Identifizieren des Probenbehälters (40).

9. Erntemaschine (10) mit einer Vorrichtung nach einem der vorhergehenden Ansprüche, insbesondere Feldhäcksler.

10. Erntemaschine nach Anspruch 9, **dadurch gekennzeichnet, dass** die Leiteinrichtung im Austragschacht (26) der Erntemaschine (10) angeordnet ist.
